## Europäisches Patentamt
## European Patent Office
## Office européen des brevets

(19)

(11) Numéro de publication : **0 146 420 B1**

(12)

# FASCICULE DE BREVET EUROPÉEN

(45) Date de publication du fascicule du brevet :
06.05.87

(51) Int. Cl.⁴ : **C 12 N 1/04, C 12 M 1/12**

(21) Numéro de dépôt : 84402012.3

(22) Date de dépôt : 08.10.84

(54) Procédé de préparation de concentrés de microorganismes lyophilisés.

(30) Priorité : 27.10.83 FR 8317156

(43) Date de publication de la demande :
26.06.85 Bulletin 85/26

(45) Mention de la délivrance du brevet :
06.05.87 Bulletin 87/19

(84) Etats contractants désignés :
AT BE CH DE FR GB IT LI LU NL SE

(56) Documents cités :
EP-A- 0 065 895
DE-A- 2 813 714
FR-A- 2 126 452
US-A- 3 911 140

(73) Titulaire : L'AIR LIQUIDE, SOCIETE ANONYME POUR
L'ETUDE ET L'EXPLOITATION DES PROCEDES
GEORGES CLAUDE
75, Quai d'Orsay
F-75321 Paris Cedex 07 (FR)

(72) Inventeur : Amen, Jean
108, bd de la Reine
F-78000 Versailles (FR)
Inventeur : Cabau, Michel
Le Plan du Guillou
F-38500 Coublevie-Voiron (FR)

(74) Mandataire : Leclercq, Maurice et al
L'AIR LIQUIDE SOCIETE ANONYME POUR L'ETUDE
ET L'EXPLOITATION DES PROCEDES GEORGES
CLAUDE 75, Quai d'Orsay
F-75321 Paris Cedex 07 (FR)

**0 146 420**

## Description

La présente invention concerne un procédé de préparation de concentrés de microorganismes lyophilisés, notamment bactéries lactiques, du genre où l'on alimente un milieu de culture, préalablement ensemencé, et maintenu à une température comprise entre 25 °C et 45 °C, de préférence de l'ordre de 30 °C, en substrat nutritif comportant du lactose et de la levure, en agent neutralisant, notamment ammoniaque ou soude, destiné à maintenir le pH du milieu de culture à une valeur constante comprise entre 5,5 et 7,5, en particulier entre 6 et 7, le tout dilué dans de l'eau, l'on règle les apports en substrat et en eau par rapport à l'agent neutralisant et cela par détermination permanente de la concentration en agent inhibiteur, de façon à assurer dans une première phase un taux de croissance élevé jusqu'à atteindre une concentration critique de l'agent inhibiteur, qui correspond à une concentration maximale admissible pour un taux de croissance plus modéré compris entre 0,10 et 0,50 m choisi pour une phase ultérieure de croissance, et l'on met en œuvre une opération d'ultrafiltration d'élimination partielle de l'agent inhibiteur avec maintien d'un volume constant du milieu de culture.

L'objet de l'invention est de spécifier les modalités indispensables des opérations qui suivent celle de la culture et qui permettent d'assurer une lyophilisation optimale.

Selon l'invention, on procède aux opérations suivantes :

a) on met fin à l'alimentation des apports en substrat nutritif, et eau, tout en maintenant la régulation du pH à ladite valeur constante par admission d'agent neutralisant et,

b) on poursuit l'opération d'ultrafiltration simultanément à un refroidissement de façon à assurer une concentration primaire en microorganismes comprise entre 40 et 80 kg poids sec bactérien par $m^3$, à une température comprise entre 15 °C et 20 °C, de préférence de l'ordre de 16 °C.

c) on procède au lavage du concentré primaire, au moyen d'une solution de lavage constituée d'un mélange de sels minéraux et d'acides aminés, oligoéléments et vitamines ;

d) on assure toujours par ultrafiltration une concentration finale comprise entre 90 et 220 kg poids sec bactérien/$m^3$, en maintenant la température telle qu'indiquée en b) ;

e) on refroidit le concentré bactérien à une température de l'ordre de 5 °C et l'on y ajoute un protecteur concentré ;

f) on procède à une congélation du concentré bactérien et à sa lyophilisation.

A titres d'exemples, la composition de la solution de lavage peut être choisie parmi celles énumérées ci-dessous :

### Exemple 1 :

| | |
|---|---|
| E. levure | 15-25 g/l |
| $KH_2PO_4$ | 3-5 g/l |
| $Na_2HPO_4$, 12 $H_2O$ | 20-30 g/l |
| $MgSO_4$, 7$H_2O$ | 0,3-0,8 g/l |

### Exemple 2 :

| | |
|---|---|
| E. levure | 1-3 g/l |
| $KH_2PO_4$ | 1-4 g/l |
| $Na_2HPO_4$, 12 $H_2O$ | 7-13 g/l |
| $MgSO_4$, 7 $H_2O$ | 0,3-0,6 g/l |
| ClNa | 8-12 g/l |

### Exemple 3

| | |
|---|---|
| E. levure | 1-3 g/l |
| $KH_2PO_4$ | 1-2 g/l |
| $Na_2HPO_4$, 12 $H_2O$ | 4-8 g/l |
| $MgSO_4$, 7 $H_2O$ | 0,3-0,6 g/l |
| ClNa | 8-12 g/l |
| Glycérophosphate Na | 3-8 g/l |

### Exemple 4

| | |
|---|---|
| E. levure | 1-3 g/l |
| $KH_2PO_4$ | 1-2 g/l |
| $Na_2HPO_4$, 12 $H_2O$ | 4-8 g/l |
| $MgSO_4$, 7 $H_2O$ | 0.3-0,6 g/l |
| ClNa | 6-10 g/l |
| Glycérophosphate Na | 3-8 g/l |

2

| Glutamate Na | 2-5 g/l |
|---|---|

Exemple 5

| E. levure | 1-3 g/l |
|---|---|
| $KH_2PO_4$ | 1-2 g/l |
| $Na_2HPO_4$, 12 $H_2O$ | 4-8 g/l |
| $MgSO_4$, 7 $H_2O$ | 0,3-0,6 g/l |
| ClNa | 5-9 g/l |
| Glycérophosphate Na | 3-8 g/l |
| Glutamate Na | 2-5 g/l |
| Ascorbate de Sodium | 2-5 g/l |

L'ensemble de ces mesures mettent en œuvre successivement un maintien d'un pH constant entre 5,5 et 7,5 et plus particulièrement entre 6 et 7, un refroidissement et une concentration primaire spécifiques, puis un lavage et une concentration finale, permettant d'assurer, comme l'expérience seule peut le prouver, une lyophilisation parfaite des microorganismes, en particulier des bactéries lactiques.

**Revendications**

1. Procédé de préparation de concentrés de microorganismes lyophilisés, notamment bactéries lactiques, du genre où l'on alimente un milieu de culture, préalablement ensemencé, et maintenu à une température comprise entre 25 °C et 45 °C, de préférence de l'ordre de 30 °C, en substrat nutritif comportant du lactose et de la levure, en agent neutralisant, notamment ammoniaque ou soude, destiné à maintenir le pH du milieu de culture à une valeur constante comprise entre 5,5 et 7,5 en particulier entre 6 et 7, le tout dilué dans de l'eau, l'on règle les apports en substrat et en eau par rapport à l'agent neutralisant et cela par détermination permanente de la concentration en agent inhibiteur, de façon à assurer dans une première phase un taux de croissance élevé jusqu'à atteindre une concentration critique de l'agent inhibiteur, qui correspond à une concentration maximale admissible pour un taux de croissance plus modéré compris entre 0,10 et 0,50 choisi pour une phase ultérieure de croissance, et l'on met en œuvre une opération d'ultrafiltration d'élimination partielle de l'agent inhibiteur avec maintien d'un volume constant du milieu de culture, caractérisé en ce que, l'opération de développement de microorganismes étant terminée, on procède aux opérations suivantes :

a) on met fin à l'alimentation des apports en substrat nutritif et eau, tout en maintenant la régulation du pH à ladite valeur constante par admission d'agent neutralisant et,

b) on poursuit l'opération d'ultrafiltration simultanément à un refroidissement de façon à assurer une concentration primaire en microorganismes comprise entre 40 et 80 kg poids sec bactérien par $m^3$, à une température comprise entre 15 °C et 20 °C, de préférence de l'ordre de 16 °C ;

c) on procède au lavage du concentré primaire, au moyen d'une solution de lavage constituée d'un mélange de sels minéraux et d'acides aminés ;

d) on assure toujours par ultrafiltration une concentration finale comprise entre 90 et 220 kg poids sec bactérien/$m^3$ ;

e) on refroidit le concentré bactérien à une température de l'ordre de 5 °C et l'on y ajoute un protecteur concentré ;

f) on procède à une congélation du concentré bactérien et à sa lyophilisation.

2. Procédé de fabrication de microorganismes selon la revendication 1, caractérisé en ce que la solution de lavage comprend un extrait de levure, $KH_2 PO_4$, $Na_2H PO_4$, 12 $H_2O$, $MgSO_4$, 7$H_2O$.

3. Procédé de fabrication de microorganismes selon la revendication 2, caractérisé en ce que la solution de lavage comprend du Cl Na.

4. Procédé de fabrication de microorganismes selon la revendication 3, caractérisé en ce que la solution de lavage comprend du Glycérophosphate de sodium.

5. Procédé de fabrication de microorganismes selon la revendication 4, caractérisé en ce que la solution de lavage comprend du Glutamate de sodium.

6. Procédé de fabrication de microorganismes selon la revendication 5, caractérisé en ce que la solution de lavage comprend de l'Ascorbate de Sodium.

**Claims**

1. Process for preparing lyophilized concentrates of microorganisms, especially lactic bacteria, feeding into a cultural medium, previously inoculated and maintained at a temperature between 25 °C and 45 °C, preferably in the order of 30 °C, a nutrient substrate comprising lactose and yeast, and a neutralizing agent, especially ammonia or soda, destined to maintain the pH of the cultural medium at a constant value between 5,5 and 7,5, especially between 6 and 7, all being diluted by water, the additions of

3

substrate and of water being regulated with regard to the neutralizing agent by permanent determination of the concentration of inhibitor agent to ensure in a first phase an elevated degree of growth until a critical concentration of the inhibitor agent is reached which corresponds to a maximum concentration admissible for a weaker degree of growth between 0,1 and 0,50 selected for a later phase of growth, and carrying out an ultra filtration for partial elimination of the inhibitor agent maintaining a constant volume of the culture medium, characterized in that on termination of the development of microorganisms one proceeds with the following operations :

a) one terminates feeding the additions of nutrient substrate and water maintaining the regulation of pH at the said constant value by admission of a neutralizing agent and

b) one carries out the ultra filtration simultaneously with a refrigeration to ensure a primary concentration of microorganisms between 40 and 80 kg bacterial dry weight per m³ at a temperature between 15 °C and 20 °C, preferably in the order of 16 °C,

c) one washes the primary concentrate by means of a washing solution consisting of a mixture of mineral salts and amino acids,

d) one ensures always by ultra filtration a final concentration between 90 and 220 kg bacterial dry weight/m³,

e) one refrigerates the bacterial concentrate at a temperature in the order of 5 °C and adds a protector concentrate,

f) one congeals the bacterial concentrate and lyophilizes it.

2. Process for preparing microorganisms according to claim 1, characterized in that the washing solution comprises a yeast extract, $KH_2PO_4$, $Na_2HPO_4 \cdot 12H_2O$ and $MgSO_4 \cdot 7H_2O$.

3. Process for preparing microorganisms according to claim 2, characterized in that the washing solution contains NaCl.

4. Process for preparing microorganisms according to claim 3, characterized in that the washing solution contains sodium glycerophosphate.

5. Process for preparing microorganisms according to claim 4, characterized in that the washing solution contains sodium glutamate.

6. Process for preparing microorganisms according to claim 5, characterized in that the washing solution contains sodium ascorbate.

## Patentansprüche

1. Verfahren zur Herstellung von lyophilisierten Konzentraten von Mikroorganismen, insbesondere von Milchbakterien, wobei man einem Kulturmedium, welches vorher beimpft wurde und auf einer Temperatur zwischen 25 °C und 45 °C, vorzugsweise in der Größenordnung von 30 °C gehalten wird, ein Laktose und Hefe enthaltendes Nährsubstrat und ein Neutralisiermittel, insbesondere Ammoniak oder Soda, das dazu bestimmt ist, den pH-Wert des Kulturmediums auf einem konstanten Wert zwischen 5,5 und 7,5, besonders zwischen 6 und 7 zu halten, zusetzt, wobei das Ganze mit Wasser verdünnt ist, wobei man die Zugaben an Substrat und an Wasser in Bezug auf das Neutralisiermittel steuert, und zwar durch ständige Bestimmung der Konzentration an Hemmittel, um in einer ersten Phase einen erhöhten Wachstumsgrad zu gewährleisten, bis eine kritische Konzentration des Hemmittels erreicht ist, die einer Maximalkonzentration entspricht, welche für einen schwächeren Wachstumsgrad zwischen 0,10 und 0,50, der für eine spätere Wachstumsphase ausgewählt wird, zulässig ist, und wobei man eine Ultrafiltration zur Teilentfernung des Hemmittels unter Aufrechterhaltung eines konstanten Volumens des Kulturmediums durchführt, dadurch gekennzeichnet, daß man bei Beendigung der Entwicklung der Mikroorganismen folgende Verfahrensmaßnahmen durchführt :

a) man beendet die Einführung der Zugaben an Nährsubstrat und Wasser, wobei man die Regulierung des pH-Wertes auf dem besagten konstanten Wert durch Zugabe von Neutralisiermittel aufrechterhält, und

b) führt die Ultrafiltration gleichzeitig mit einer Kühlung dergestalt, daß eine Primärkonzentration an Mikroorganismen zwischen 40 und 80 kg Bakterientrockengewicht/m³ gewährleistet ist, bei einer Temperatur zwischen 15 °C und 20 °C, vorzugsweise in der Größenordnung von 16 °C, durch,

c) man wäscht das Primärkonzentrat mit einer Waschlösung, die aus einem Gemisch von Mineralsalzen und Aminosäuren besteht,

d) man gewährleistet immer durch Ultrafiltration eine Endkonzentration zwischen 90 und 220 kg Bakterientrockengewicht/m³,

e) man kühlt das Bakterienkonzentrat auf eine Temperatur in der Größenordnung von 5 °C und setzt ihm ein Schutzkonzentrat zu,

f) man gefriert das Bakterienkonzentrat und lyophilisiert es.

2. Verfahren zur Herstellung von Mikroorganismen nach Anspruch 1, dadurch gekennzeichnet, daß die Waschlösung ein Hefeextrakt, $KH_2PO_4$, $Na_2HPO_4 \cdot 12H_2O$ und $MgSO_4 \cdot 7H_2O$ enthält.

3. Verfahren zur Herstellung von Mikroorganismen nach Anspruch 2, dadurch gekennzeichnet, daß die Waschlösung NaCl enthält.

4. Verfahren zur Herstellung von Mikroorganismen nach Anspruch 3, dadurch gekennzeichnet, daß die Waschlösung Natriumglycerophosphat enthält.

5. Verfahren zur Herstellung von Mikroorganismen nach Anspruch 4, dadurch gekennzeichnet, daß die Waschlösung Natriumglutamat enthält.

6. Verfahren zur Herstellung von Mikroorganismen nach Anspruch 5, dadurch gekennzeichnet, daß die Waschlösung Natriumascorbat enthält.